Europäisches Patentamt

European Patent Office

Office européen des brevets

(19)

(11) **EP 1 138 330 A1**

(12) ## EUROPEAN PATENT APPLICATION

(43) Date of publication:
**04.10.2001 Bulletin 2001/40**

(51) Int Cl.⁷: **A61K 31/485**
// (A61K31/485, 31:24)

(21) Application number: **99125752.8**

(22) Date of filing: **23.12.1999**

(84) Designated Contracting States:
**AT BE CH CY DE DK ES FI FR GB GR IE IT LI LU MC NL PT SE**
Designated Extension States:
**AL LT LV MK RO SI**

(71) Applicant: **WARNER-LAMBERT COMPANY**
**Morris Plains New Jersey 07950 (US)**

(72) Inventors:
• **Hamon, Jacques**
  **91400 Orsay (FR)**

• **Roman, François**
  **94400 Vitry-sur-Seine (FR)**

(74) Representative:
**Dufresne, Guillaume Alain François**
**Warner-Lambert Company/Parke Davis**
**Pfizer Global Research & Development**
**Fresnes Laboratories/Service Brevets**
**3-9, rue de la Loge/B.P. 100**
**94265 Fresnes Cédex (FR)**

(54) **Combination of trimebutine with an opioid analgesic**

(57)     The invention provides a combination of of tri-mebutine [2-dimethylamino-2-phenylbutyl-3, 4, 5- trimethoxy-benzoate hydrogen maleate] or its corresponding stereoisomers with an opioid analgesic for the preparation of a medicament to prevent and/or treat pain or nociception.

EP 1 138 330 A1

**Description**

FIELD OF THE INVENTION

The field of the invention is related to methods for preventing and/or treating pain. More particularly the invention concerns a combination of trimebutine [2-dimethylamino-2-phenylbutyl-3, 4, 5- trimethoxy-benzoate hydrogen maleate] or its corresponding stereoisomers with an opioid analgesic for preventing and/or treating pain as well as nociception .

BACKGROUND OF THE INVENTION

Trimebutine [2-dimethylamino-2-phenylbutyl 3, 4, 5- trimethoxybenzoate hydrogen maleate; TMB] has been used in many countries since 1969 for the treatment of functional bowel disorders, including irritable bowel syndrome (IBS). The efficacy of the compound to relieve abdominal pain has been demonstrated in various clinical studies using different protocols of treatment (Lüttecke, 1980 ; Moshal and Herron, 1979, Toussaint *et al.,* 1981 ; Ghidini *et al.* 1986). Trimebutine was found to display weak agonist activity for rat brain and guinea-pig (Roman *et al.,* 1987) or canine (Allescher *et al.,* 1991) intestinal opioid receptors, without selectivity for any of the $\mu$-, $\delta$- and K-subtypes. This weak activity was confirmed when using isolated intestinal fragments under transmural stimulation (Pascaud *et al.,* 1987). This property could be responsible for the modulatory action of trimebutine on intestinal motility in fasted dog. Trimebutine given either intravenously or orally delays the appearance of a phase III of the migrating motor complex (MMC) in the stomach and the duodenum by inducing a premature phase III, migrating along the whole intestine (Bueno *et al.,* 1987). In man, trimebutine stimulates intestinal motility in both fed and fasted states (Grandjouan *et al*., 1989). Furthermore, trimebutine reverses the effect of stress in jejunal motility (Delis *et al.,* 1994).

More recently, trimebutine has been shown able to influence the activity of visceral afferents by decreasing the intensity of the recto-colonic reflex in rats as evidenced by the inhibition of colonic motility consecutive to rectal distension (Julia *et al.,* 1996). This result may be related to the beneficial effects found with trimebutine in patients with IBS and more specifically in the treatment of attacks of abdominal pain.

Pain has been defined as the sensory experience perceived by nerve tissue distinct from sensation like touch, pressure, heat or cold.

Although the precise definition of pain is difficult because of the large range of sensations as well as the variation in pain perception by different individuals, it can be classified in three categories : acute pain, inflammatory pain and chronic pain.

Generally speaking, physicians and patients are expecting more efficient and safe compounds for treating and/or preventing pain. It is the major case for chronic pain, where there is a general agreement (9th World Congress on Pain, Vienna, August 1999) that there is an unmet medical need for its treatment. NSAIDs and opiates are quite ineffective in many cases. Antidepressants are being used with inconsistent eficacy (50-60%). Certain anticonvulsants (carbamazepine, clonazepam, baclofen) may be active. In extreme cases, capsaicin and local anesthetics are being tried. However, none of these approaches is satisfactory and some patients are refractory to all of them. In some cases like trigeminal neuralgia, neurosurgery (differential thermocoagulation of Gasser ganglion) remains the only way of alleviating pain.

In this view, clinical and animal studies have shown that concomittant opiate and bupivacaine therapy improves the magnitude and duration of pain relief when compared to the effects of each drug administered separately (Meert and Melis, 1992).

In addition, since NMDA (N-methyl-D-aspartate)-type glutamate receptors are believed to play a pivotal role in the transmission of excitatory signals from primary sensory neurones to the brain through the spinal cord (Dickenson et al., 1990), some strategies have been set up for the treatment of pain by administration of a combination of NMDA antagonists and an opioid analgesic (WO 99/44610). Unfortunately, some products as dextromethorphan have been reported to induce unacceptable side effects.

From a starting point in visceral pain, the inventors found, as confirmed in the present application, that trimebutine has an inhibitory action on glutamate release through the blockade of sodium channels and also is able to act as a local anesthesic with a 17-fold higher potency than lidocaine. Surprisingly, inventors have shown that trimebutine or its stereoisomers, which is a non-NMDA receptors antagonist, administred in combination with an opioid analgesic on one hand has an inhibitory action on pain with a potentiation of opioid analgesic action and on the other hand reduces the unwanted side-effects of the said opioid analgesic among them drug dependence and constipation. Indeed, there is no general teaching in the art suggesting a combination of trimebutine or its stereoisomers with an opioid analgesic.

According to these results, it is demonstrated that trimebutine can have an action on pain conditions other than visceral pain, confirming that trimebutine is useful in the treatment and/or the prevention of pain.

SUMMARY OF THE INVENTION

The invention relates to a product comprising trimebutine [2-dimethylamino-2-phenylbutyl-3, 4, 5- trimethoxy-benzoate hydrogen maleate] or its corresponding stereoisomers and an opioid analgesic. The components of the said product can be used simultaneously, separately or sequentially, as a combined preparation, in the treatment and/or prevention of pain and/or nociception.

In this regard, the opioid analgesic is preferably morphine but can also be chosen among codeine, dihydrocodeine, diacethylmorphine, hydrocodone, hydromorphone, levorphanol, onyxmorphone, alfentanyl, buprenorphine, butorphanol, fentanyl, sulfentanyl, meperidine, methadone, nalbuphine, propoxyphene and pentazocine or acceptable salts thereof.

The invention provides also a pharmaceutical composition comprising trimebutine or its corresponding stereoisomers and an opioid analgesic with at least one pharmaceutical carrier or excipient.

Another embodiment of the invention is the use of trimebutine or its corresponding stereoisomers and an opioid analgesic for the preparation of a medicament which comprises both trimebutine and the said opioid analgesic in a single dosage form or as a separate dosage forms which can be used simultaneously, separately or sequentially in the treatment and/or prevention of pain and/or nociception. In this respect, the present invention is useful to prevent and/or treat acute pain, inflammatory pain and/or chronic pain.

In the context of the invention, trimebutine is administered at dosage between 50 to 900 mg/day and preferably between 300 to 600 mg/day and the opioid analgesic is administered at dosage between 0.01 to 250 mg/day depending of the chosen opioid analgesic.

Preferably, the opioid analgesic is morphine which is administered at dosage between 2 to 220 mg/day.

The present application concerns also a method for preventing and/or treating pain and/or nociception comprising administering trimebutine and an opioid analgesic to a patient in need thereof. The administration of trimebutine and the opioid analgesic may be simoultaneous, separate or sequential.

The invention relates also to a process for the preparation of a pharmaceutical composition comprising trimebutine or its stereoisomers and an opioid analgesic, which process comprises bringing trimebutine and the opioid analgesic into association with a pharmaceutically acceptable carrier or excipient.

DESCRITION OF DRAWINGS

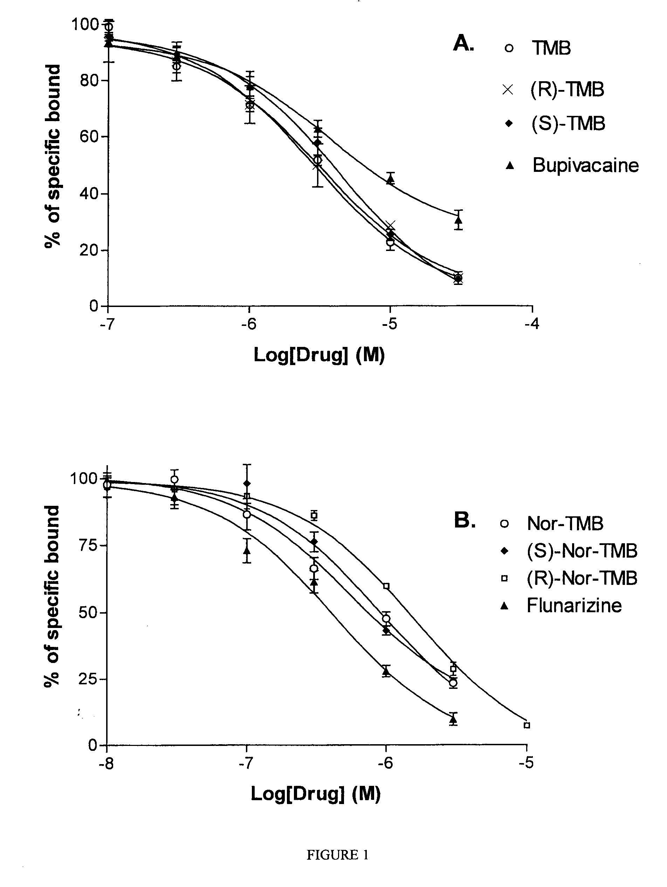

**Figures 1 :** Effect of TMB **(A)**, Nor-TMB **(B)** and their corresponding stereoisomers on [$^3$H]-batrachotoxin binding to rat cortical synaptosomes. Membranes are incubated with increasing concentrations of test drugs in presence of 25 μg scorpion venom and 10 nM [$^3$H]-batrachotoxin. Non specific binding is determined in the presence of 0.3 mM veratridine. After 90 min incubation at 25°C, bound ligand is separated from free ligand by vacuum filtration through GF/B filters. Specific binding in presence of test compounds is calculated as percentage of control binding determined in absence of inhibitors. Represented values are mean ± SEM from at least 3 independent determinations performed in duplicate.

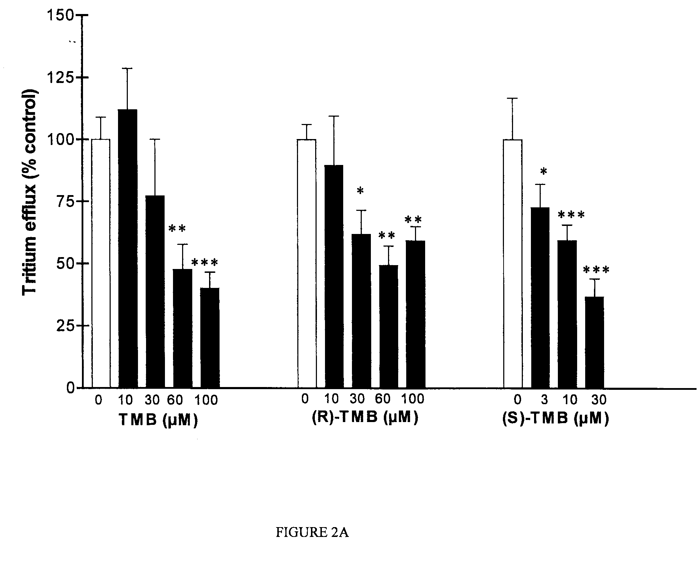

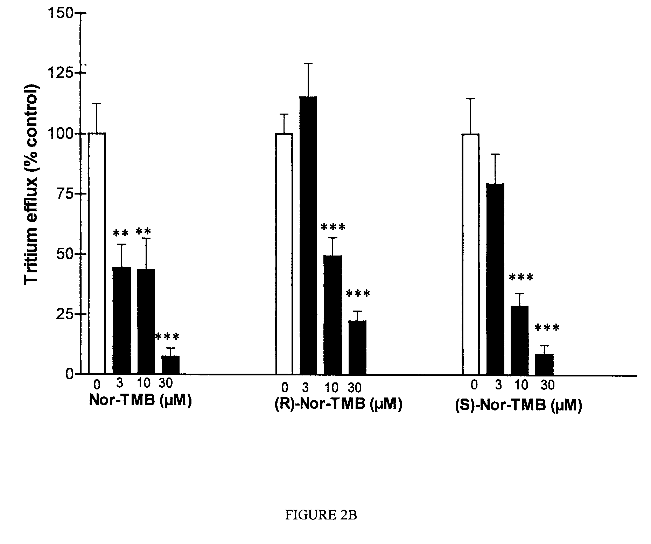

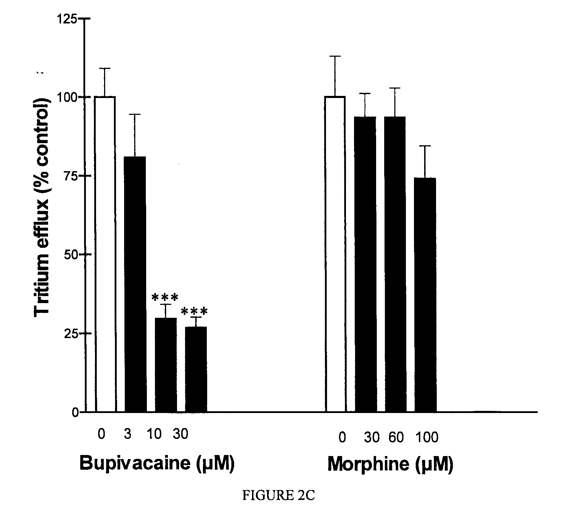

**Figure 2 :** Effect of TMB **(A)**, Nor-TMB **(B)** and their corresponding stereoisomers, on veratridine-induced glutamate release from rat spinal cord slices. Morphine and bupivacaine **(C)** are tested in the same conditions. Results are means ± SEM of at least 10 determinations. The slices are superfused 15 min with the test compound prior to stimulation with veratridine (40 μM) . The radioactivity collected in 5 min fractions during 30 min after the stimulation is counted and the effect of compound is determined by comparing the cumulated quantity of radioactivity released to that obtained in cells superfused with buffer alone.*P<0.05; **P<0.01;***P<0.001, Student's test.

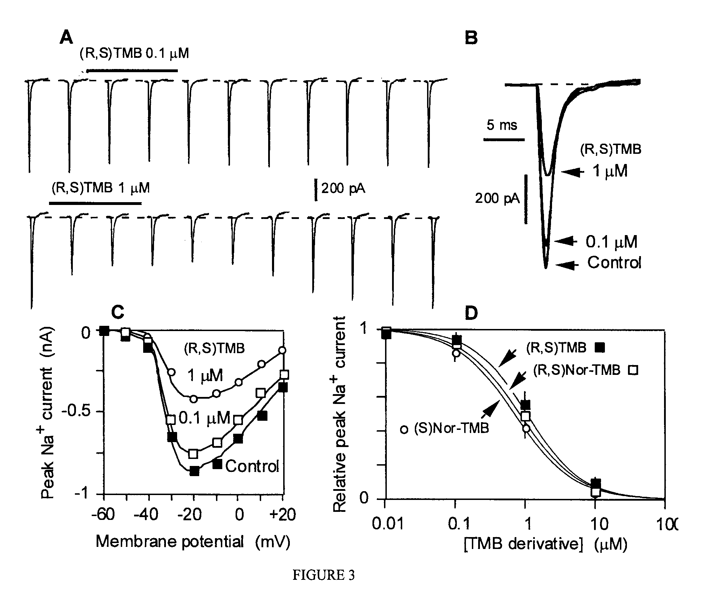

**Figure 3 :** Effect of TMB on sodium currents measured in DRG neurons. **(A)** Inward Na$^+$ current induced every 10 s by stepping the membrane potential from -80 to -10 mV. TMB is locally applied for 20 s at 0.1 μM (top row) and at 1 μM (bottom row). **(B)** Sodium current before (control) and during TMB perfusion (same cell as in A.). **(C)** Peak sodium current versus pulse potential in control saline and in the presence of TMB at the concentrations indicated. The decrease in peak sodium current occurred homothetically. **(D)** Dose-response relationship of TMB effects on DRG Na$^+$ current. Results are expressed as the Na$^+$ current part (relative peak Na$^+$ current) persisting in the presence of the blocker. Each point is mean ± S.E.M. of 4 to 6 experiments. Continuous curve: best fit to Hill function with IC$_{50}$ = 0.69 μM, and n$_H$ = 1.02.

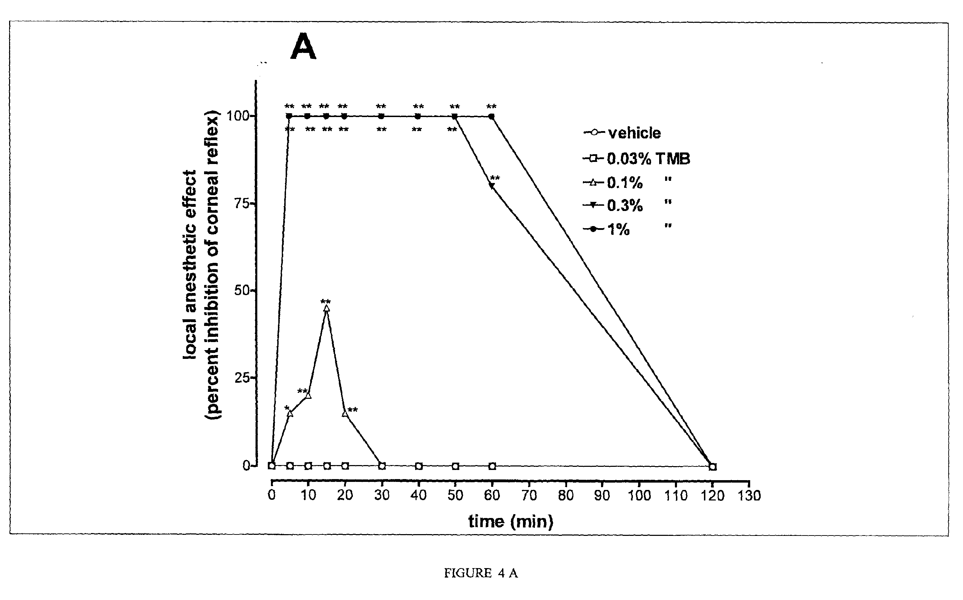

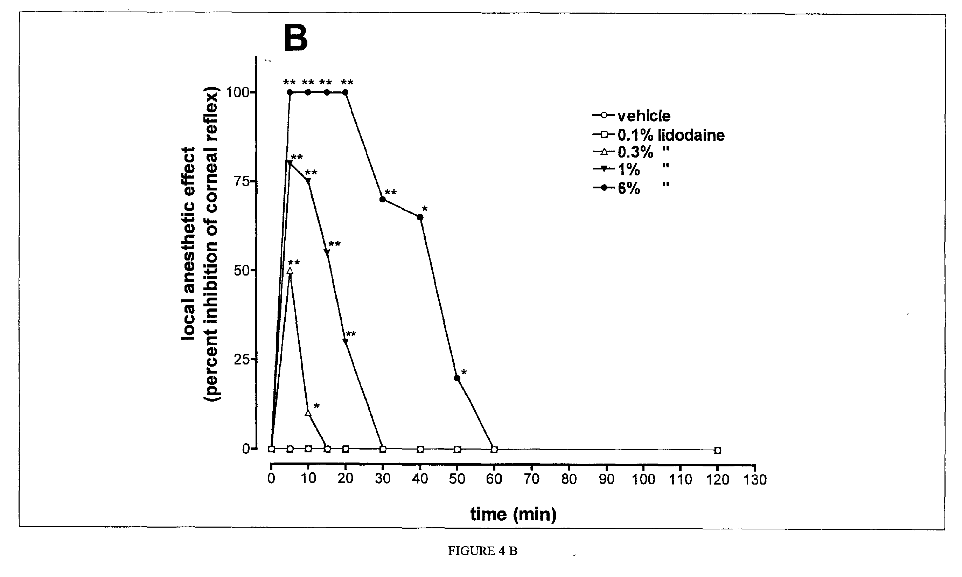

**Figure 4 :** Local anesthetic effect of TMB **(A)** and lidocaine **(B)** as determined in the rabbit corneal reflex. The values represent the median of percentage of inhibition of corneal reflex calculated for each set of 10 stimulations. The x axis represents time (in minutes) following instillations.*P<0.05 ; **P<0.01 compared with the control group treated with the vehicle (Mann-Whitney U test).

DETAILED DESCRIPTION OF THE INVENTION

After having determined that trimebutine is able to inhibit the glutamate release involved in the transmission of nociceptive stimulus, through the blockade of sodium channels, the inventors have confirmed that trimebutine is able to act as local anesthesic and surprisingly with a potency really higher than of lidocaine. They have also established that a combined administration of trimebutine or its steroisomers with an opioid analgesic has a potentiating effect for treating and /or preventing pain.

In this regard, the invention provides a product comprising trimebutine [2-dimethylamino-2-phenylbutyl-3, 4, 5- trimethoxy-benzoate hydrogen maleate] or its corresponding stereoisomers and an opioid analgesic in order to answer the need of pain relief by an always more efficient and safe compound .

In this respect, the components of the said product can be used simultaneously, separately or sequentially in the treatment and/or prevention of pain and/or nociception.

Trimebutine (2-dimethylamino-2-phenylbutyl 3, 4, 5- trimethoxybenzoate hydrogen maleate, TMB) has been demonstrated to be active for relieving abdominal pain in humans.

Furthermore, it is known that in humans, after oral or iv administration of trimebutine , this latter serves as a metabolic precursor for N-desmethyl trimebutine (Nor-TMB). This indicates that trimebutine is acting like a bioprecursor of Nor-TMB meaning that under action of the hepatic enzymes the bioprecursor trimebutine is metabolized and gives rise to a new molecule.

Indeed, the administration of trimebutine in humans leads to the concomittant exposure of trimebutine, Nor-TMB and other metabolites. Therefore these compounds and particularly trimebutine and Nor- TMB are able to elicit jointly their (antinociceptive) properties.

It is therefore understood that N-desmethyl trimebutine (Nor-TMB) comprises the stereoisomers (S) N-desmethyl trimebutine and (R) N-desmethyl trimebutine and the corresponding racemate.

The opioid analgesics are well established as a class of analgesic agent. Sometimes they are also referred to as opiates but this term should be reserved for morphine-related chemicals. The term opioid is generally accepted to refer in a generic sense to all drugs, natural or synthetic, with morphine-like actions.

The synthetic or semi-synthetic opioid analgesics are derivatives of five chemical classes of compounds : phenanthrenes, phenylheptylamines, phenylpiperidines, morphinans and benzomorphans.

In the context of the invention, the opioid analgesic is chosen among morphine, codeine, dihydrocodeine, diacethylmorphine, hydrocodone, hydromorphone, levorphanol, onyxmorphone, alfentanyl, buprenorphine, butorphanol, fentanyl, sulfentanyl, meperidine, methadone, nalbuphine, propoxyphene and pentazocine or acceptable salts thereof. Preferably, the opioid analgesic is morphine or an acceptable salt thereof.

Of all of the opioid analgesics, morphine remains the most widely used and in this regard is an archetype compound. Unfortunately, apart from its useful therapeutic properties, morphine also has a number of drawbacks including sedation, respiratory depression, decreased gastrointestinal motility (resulting in constipation) and in some individuals, nausea and vomiting may occur. Another drawback always reported is the developpment of tolerance and physical dependence limiting the clinical use of such a compound. There is therefore a need to develop methods which enable the clinicians to use lower doses of opioid analgesics such as morphine, thereby reducing the likelihood of adverse effects including the development of tolerance and dependence, and thus avoiding two major problems : the need to escalate dosages in long term treatment cases leading to the occurrence of side effects and the risk of drug withdrawal associated with cessation of administration.

The present invention also concerns advantageously the use of trimebutine [2-dimethylamino-2-phenylbutyl-3, 4, 5- trimethoxy-benzoate hydrogen maleate] or its corresponding stereoisomers and an opioid analgesic for the preparation of a medicament to prevent and/or treat pain and/or nociception. Accordingly, the compounds of the combined preparation are either used simultaneously, separately or sequentially in the treatment and/or prevention of pain and/or nociception. It is understood that the term "pain" comprises any kind of pain with any etiology.

More particularly, the invention is useful for preventing and/or treating acute pain as well as any inflammatory pain (visceral or somatic) and chronic pain.

Interestingly, the inventors have established that trimebutine and also its metabolites called hereafter Nor-TMB (and preferably the (S)-enantiomer) which is one of its major metabolites in humans, inhibit glutamate release from rat spinal cord slices, through the blockade of sodium channels. Especially, trimebutine, its stereoisomers, and Nor-TMB have been studied for their affinity towards sodium channels labeled by [3H]-batrachotoxin, their effect on sodium currents in rat dorsal root ganglia neurons, on veratridine-induced glutamate release from rat spinal cord slices.

These results are particularly interesting since the pivotal role of glutamate and excitotoxic amino-acids (EAA) in the establishment of hyperalgesic or allodynic conditions has been evidenced by Dickenson et al., 1995. From that discovery, strategies have been set up for finding new analgesic drugs based on the inhibition of glutamate and EAA receptors. Most of the inhibitors that these strategies have generated cannot be used in human for safety reasons and it appears now that the blockade of glutamate receptors is a difficult way for drug discovery.

Furthermore, the results reported in the examples demonstrate that trimebutine, its stereoisomers and its metabolites display an analgesic activity and an inhibitory effect on pain, particularly on inflammatory pain and chronic pain.

Examples of acute pain include particularly post-operative pain and headaches.

Inflammatory pain includes any pain with an inflammatory component such as arthritis, polyarthritis, spondylarthritis, musculo-squeletal pain such as osteo-traumatic pain, as well as phantom limb pain, back pain, vertebral pain, shipped disc surgery failure, post-surgery pain, rheumatic pain, dental pain and dysmenorrhoea.

Chronic pain, according to the definition proposed by the International Association for the Study of Pain, is a pain which persists beyond normal tissue healing time (suggested three months) (International Association for the Study of Pain, classification of chronic pain. Pain, 1986, Suppl 3, S1-S226), and this implies a transition point from acute pain.

Accordingly and since chronic pain results from hyperalgesia (Dickenson et al., 1995), the present invention comprises also the prevention and/or treatment of hyperalgesia or pain related to central hypersensitivity conditions.

Examples of chronic pain include neurological pain such as neuropathies, polyneuropathies including those related to diabetes, headache, trauma, neuralgias including post-zosterian neuralgia and trigeminal neuralgia, algodystrophy, HIV-related pain, cancer-related pain, vascular pain such as pain resulting from Raynaud's syndrome, Horton's disease, arteritis, varicose ulcers.

In the context of the invention, trimebutine is administered at dosage between 50 to 900 mg/day and preferably between 300 to 600 mg/day (man with average weigth of 70 kg) .

The opioid analgesic is administered at a dosage level up to conventional dosage levels for such analgesic, but preferably at a reduced level in accordance with the instant invention. Suitable dosage levels will depend upon the analgesic effect of the chosen opioid analgesic but typically suitable dosage levels are between about 0.001 to 25 mg/kg per day, preferably between 0.005 to 10 mg/kg per day and especially between 0.005 to 5 mg/kg per day or between 0.01 to 250 mg/day (man with average weigth of 70 kg) depending of the chosen opioid analgesic. The compound may be adminsitred up to 6 times per day and preferably 1 to 4 times per day.

For example, dosages may be for morphine preferably between 2 to 220 mg/day, for codeine between 20 to 60 mg/day, for fentanyl betweeen 0.1 to 1 mg/24 hours, droperydol between 2 to 10 mg/day, for oxycodone between 20 to 80 mg every 4 hours (man with average weigth of 70 kg).

When administered in combination, either as a single or as separate pharmaceutical composition(s), trimebutine or its stereoisomers and the opioid analgesic are presented in a ratio which is consistent with the manifestation of the desired effect. In particular, the ratio by weight of trimebutine to the opioid analgesic will suitably be approximately 1 to 1. Preferably this ratio will be between 0.0001 to 1 and 1000 to 1 and especially between 0.01 to 1 and 100 to 1.

In a further aspect of the invention, there is provided a pharmaceutical composition comprising trimebutine or its corresponding stereoisomers and an opioid analgesic.

In this context, trimebutine provided in a pharmaceutical composition in combination with an opioid analgesic is useful for preventing and/or treating the above mentioned kinds of pains. Pharmaceutical compositions include trimebutine and/or its corresponding stereoisomers including their salts and is produced by formulating the active compounds in dosage unit form with at least one solid or liquid pharmaceutical acceptable carrier or excipient.

Where it is appropriate to form a salt, the pharmaceutically acceptable salts include acetate, benzenesulfonate, benzoate, bitartrate, calcium acetate, camsylate, carbonate, citrate, edetate, edisylate, estolate, esylate, fumarate, gluceptate, gluconate, glutamate, glycoloylarsanilate, hexylresorcinate, hydrabamine, hydrobromide, hydrochloride, hydrogencarbonate, hydroxynaphthoate, iodide, isethionate, lactate, lactobionate, malate, maleate, mandelate, mesylate, methylnitrate, methylsulfate, mucate, napsylate, nitrate, pamoate (embonate), pantothenate, phosphate/diphosphate, polygalacturonate, salicylate, stearate, subacetate, succinate or hemi-succinate, sulfate or hemi-sulfate, tannate, tartrate or hemi-tartrate, theoclate, triethiodide, benzathine, chloroprocaine, choline, diethanolamine, ethylenediamine, meglumine, procaine, aluminum, ammonium, tetramethyl ammonium, calcium, lithium, magnesium, potassium, sodium, and zinc. (See also "Pharmaceutical salts" by Berge S.M. *et al.* (1997) J. Pharm. Sci. **66**: 1-19, which is incorporated herein by reference.)

The salts of opioid analgesics are also prepared according to well known processes and said salts suitable for oral or parenteral routes are preferred.

Solid dosage forms for oral administration include capsules, tablets, pills, powders, and granules. In such solid dosage forms, the active components are admixed with at least one inert customary excipient (or carrier) such as sodium citrate or dicalcium phosphate or (a) fillers or extenders, as for example, starches, lactose, sucrose, glucose, mannitol, and silicic acid, (b) binders, as for example, carboxymethylcellulose, alginates, gelatin, polyvinylpyrrolidone, sucrose, and acacia, (c) humectants, as for example, glycerol, (d) disintegrating agents, as for example, agar-agar, calcium carbonate, potato or tapioca starch, alginic acid, certain complex silicates, and sodium carbonate, (e) solution retarders, as for example paraffin, (f) absorption accelerators, as for example, quaternary ammonium compounds, (g) wetting agents, as for example, cetyl alcohol, and glycerol monostearate, (h) adsorbents, as for example, kaolin and bentonite, and (i) lubricants, as for example, talc, calcium stearate, magnesium stearate, solid polyethylene glycols, sodium lauryl sulfate, or mixtures thereof. In the case of capsules, tablets, and pills, the dosage forms may also comprise buffering agents.

Solid compositions of a similar type may also be employed as fillers in soft and hard-filled gelatin capsules using such excipients as lactose as well as high molecular weight polyethyleneglycols, and the like.

Solid dosage forms such as tablets, dragées, capsules, pills, and granules can be prepared with coatings and shells, such as enteric coatings and others well known in the art. The active components can also be in micro-encapsulated form, if appropriate, with one or more of the above-mentioned excipients.

Liquid dosage forms for oral administration include pharmaceutically acceptable emulsions, solutions, suspensions, syrups, and elixirs. In addition to trimebutine and/or the opioid analgesic, the liquid dosage forms may contain inert diluents commonly used in the art, such as water or other solvents, solubilizing agents and emulsifiers, as for example, ethyl alcohol, isopropyl alcohol, ethyl carbonate, ethyl acetate, benzyl alcohol, and the like. Suspensions, in addition to trimebutine and/or the opioid analgesic, may contain suspending agents, as for example, ethoxylated isostearyl alcohols, polyoxyethylene sorbitol and sorbitan esters, microcrystalline cellulose, aluminum metahydroxide, bentonite, agar-agar and tragacanth, or mixtures of these substances, and the like.

Compositions suitable for parenteral injection may comprise physiologically acceptable sterile aqueous or non aqueous solutions, dispersions, suspensions or emulsions, and sterile powders for reconstitution into sterile injectable solutions or dispersions. Examples of suitable liquid carriers, diluents, solvents or vehicles include water, ethanol, polyols (propyleneglycol, polyethyleneglycol, glycerol, and the like), and suitable mixtures thereof.

These compositions may also contain adjuvants such as preserving, wetting, emulsifying, and dispersing agents. Prevention of the action of microorganisms can be ensured by various antibacterial and antifungal agents, for example, parabens, chlorobutanol, phenol, sorbic acid, and the like. It may also be desirable to include isotonic agents, for example sugars, sodium chloride, and the like.

Preferably the composition is in unit dosage form. In such form, the preparation is divided into unit doses containing appropriate quantities of trimebutine and/or the opioid analgesic. The unit dosage form can be a packaged preparation, the package containing discrete quantities of the preparation, for example, packeted tablets, capsules, and powders in vials or ampoules. The unit dosage form can also be a capsule, cachet, or tablet itself, or it can be the appropriate number of any of these packaged forms. Some examples of dosage unit forms are tablets, capsules, pills, powders, suppositories, aqueous and non aqueous oral solutions and suspensions, and parenteral solutions packaged in containers containing either one or some larger number of dosage units and capable of being subdivided into individual doses.

Routes of administration are preferably the oral or the parenteral routes and especially the injection routes including particularly the intravenous injection route and epidural injection route. However, any compatible route such as subcutaneous, intramuscular, intrathecal, intraperitoneal routes can also be considered in the context of the present invention.

Another embodiment of the invention is related to a method for preventing and/or treating pain and/or nociception comprising administering trimebutine and an opioid analgesic to a patient in need thereof . In the context of the invention, the administration of the two compounds may be simoultaneous, separate or sequential.

The term "patient" is intended to include any mammal and especially human whom the trimebutine and the opioid analgesic are administred.

A further embodiment of the invention provides a process for the preparation of a pharmaceutical composition comprising trimebutine or its stereoisomers and an opioid analgesic which process comprises bringing trimebutine and the opioid analgesic into association with a pharmaceutically acceptable carrier or excipient.

The biochemical and pharmacological data reported in the examples allow a better understanding of the mechanism of action of trimebutine in combination with an opioid analgesic. They support the assumption that, besides its regulatory effects on colonic motility already reported in the past and which had been related to its weak opioid properties, trimebutine is endowed with antinociceptive properties which are due to its blocking effect on $Na^+$ channels. These new properties of TMB explain how this compound in combination with an opioid analgesic is a useful method for preventing and/or treating pain.

The present invention will be further disclosed in the following examples without limiting the scope of the invention.

## EXAMPLES

**Material and Methods**

**Animals.**

Male Sprague-Dawley rats (IFFA Credo, Saint Germain sur l'Arbresle, France), weighing 225-250 g ([3H]-batrachotoxin binding experiments) or 350-375 g (glutamate release experiments), or pregnant rats (electrophysiological experiments) are used in this experiment and are cared for in accordance with the institutional guidelines for animal welfare: temperature $21 \pm 3$ °C ; light/dark: 12h/12h.

**Drugs and media.**

Trimebutine maleate, (S)-Trimebutine, (R)-Trimebutine, are synthetized according to the process disclosed in the french patent FR 2,369M (1962) and the japanese patent application published under n° 16416 (1980) and incorporated herein by reference.

Flunarizine, L-glutamatic acid, lidocaine hydrochloride, bupivacaine, trypsin and DMEM-F12 are purchased from Sigma (St Quentin Fallavier, France), morphine from Francopia (Gentilly, France), veratridine from RBI, Bioblock Scientific (Illkirch, France), gentamicine from Boehringer Mannheim S.A. (Meylan, France). All reagents used for the preparation of buffers and solutions are of analytical grade from Merck (Merck-Clevenot, Nogent sur Marne, France).

(S)-N-desmethyl-TMB maleate is synthetized according to the process disclosed in WO 99/01417 and incorporated herein by reference.

L -[ G-3H]-glutamic acid ( 49 Ci / mmole), is from Amersham (Les Ulis, France). Dulbecco's modified Eagle medium, Neurobasal medium, fetal calf serum were from Gibco, Life Technologies S.A.R.L. (Cergy Pontoise, France). Horse serum is from Seromed, (Berlin, Germany).

### Example 1 : [3H]-batrachotoxin binding.

The purpose of the present example is to determine the affinity of the tested compounds to [3H]-batrachotoxin binding sites in rat cortical synaptosomes, representing site 2 of the sodium channel

#### 1.1 Materials and methods

*a) Synaptosomal membranes.*

Cerebral cortices from male Sprague-Dawley rats are homogenized in a glass-Teflon homogenizer in 10 volumes of ice-cold 0.32 M sucrose, 5 mM $K_2HPO_4$ (pH 7.4 at 4°C). The homogenate is centrifuged at 1000g for 10 min ; the new pellet is resuspended in the same volume of sucrose and recentrifuged. The new pellet is discarded and the two supernatants resulting from these two centrifugations are pooled and centrifuged at 20,000 g for 10 min. The resulting pellet is resuspended in a sodium-free assay buffer containing 50 mM HEPES (N-2-hydroxyethylpiperazine-N'-2-ethanesulphonic acid ), 5.4 mM KCl, 0.8 mM $MgSO_4$, 5.5 mM glucose and 130 mM choline chloride (pH 7.4 at 25°C).

*b) Binding experiment.*

Binding assays are initiated by the addition of 150-200 µg synaptosomal protein to an assay buffer containing 25 µg scorpion venom (*Leireus quinquestriatus*), 0.1 % BSA, 10 nM [3H]-batrachotoxin and various concentrations of test drugs (250 µl final volume). Non-specific binding is determined in the presence of 0.3 mM veratridine. Reactions are incubated for 90 min at 25°C and the bound ligand is separated from the free by vacuum filtration through GF/B filters (Filtermate, Packard). The filters are washed with 2x5 ml buffer (5 mM HEPES, 1.8 mM $CaCl_2$, 0.8 mM $MgSO_4$, 130 mM choline chloride, 0.01% BSA; pH 7.4 at 25°C) and bound ligand is estimated by using liquid scintillation spectrometry (Topcount, Packard).

c) *Calculations.*

In all experiments examining the displacement of [3H]-batrachotoxin binding by unlabeled drugs, concentration-response curves are generated using six concentrations of drugs. All assays are performed at least three times, with each determination performed in duplicate. Data are expressed as mean values $\pm$ SEM of at least three determinations. Displacement curves are fits generated by Graph-Pad Software. Displacement plots are analysed by a non linear regression analysis using the LIGAND computer program (Mc Pherson, 1985). These analysis generated Hill coefficient ($n_H$) and $IC_{50}$ values. Ki values are calculated from $IC_{50}$ values using the Cheng-Prusoff (1973) relationship.

1.2 Results

The results presented in Figure 1 (A) and (B) show that trimebutine, its stereoisomers and its metabolites displace [3H]-batrachotoxin from its binding sites to rat cortical synaptosomes with potencies lying between that of bupivacaine (Ki = 7.14 ± 0.96) and that of flunarizine (Ki = 0.38 ± 0.05 μM). For all compounds, the displacement of [3H]-batrachotoxin is complete and the calculated Hill coefficient is close to 1 (fig.1).
The affinity of trimebutine is found with Ki = 2.66 ± 0.15 μM. For this compound, no stereoselectivity is evident since the corresponding stereoisomers display affinities similar to that of racemates. The values for the (S) and (R ) enantiomers are : Ki = 3.31 ± 0.36 μM and Ki = 2.89 ± 0.88 μM respectively.
For Nor-TMB, the values for the (S) and (R ) enantiomers are : Ki = 0.80 ± 0.04 μM and Ki = 1.26 ± 0.07 μM, respectively.
Hence, the present example demonstrates that trimebutine, Nor-TMB and the corresponding stereoisomers display affinity for [3H]-batrachotoxin binding sites in the same order of magnitude than bupivacaine or flunarizine, two sodium channel blockers.

**Example 2 : [3H]-glutamate release.**

The purpose of the present example is to determine the ability of trimebutine and its metabolites to inhibit the release of glutamate from rat spinal cord slices.
This example shows that trimebutine, its metabolites and their corresponding stereoisomers inhibit veratridine-induced glutamate release *in vitro.* Veratridine is known to induce glutamate release by activating voltage-dependent $Na^+$ channels, resulting in $Na^+$ influx with consecutive reduction of the transmembrane gradient (Wermelskirchen *et al.,* 1992).

2.1 Materials and methods

a) *Buffers.*

Two buffers are prepared: an incorporation buffer (modified Krebs solution: 119 mM NaCl, 5 mM KCl, 0.75 mM $CaCl_2$, 1.2 mM $MgSO_4$, 1 mM $NaH_2PO_4$, 25 mM HEPES, 1 mM $NaHCO_3$, 11 mM D-glucose, 67 μM EDTA, 1.1 mM L-ascorbic acid (pH 7.4) gassed with 95% $O_2$ and 5% $CO_2$) and a superfusion buffer identical to the incorporation buffer except that EDTA and ascorbic acid are omitted. Compounds to be tested and veratridine are diluted in this superfusion buffer.

b) *Rat spinal cord slices.*

After decapitation of animals, a 1.5 cm segment of lumbar spinal cord is isolated after a lumbosacral laminectomy and submerged in a ice-cold modified Krebs solution gassed with 95% $O_2$ and 5% $CO_2$. After removal of the *dura matter,* all ventral and dorsal roots are cut at the *root* of the entry zone. Slices (250 μm thick cube-like blocks) are prepared using three successive sections performed with a McIllwain tissue chopper.

c) *Superfusion experiments .*

Slices are incubated for 5 min at 30°C in 5 ml of incorporation buffer maintened under oxygenation and containing 10 μM L-glutamic acid and 4 μCi / ml [3H]-glutamic acid. After incubation, the slices are transferred into superfusion chambers in an automatic superfusion apparatus (Brandel). The apparatus consists in a device of 20 chambers allowing to run simultaneously 20 experiments and to control the sequence of buffers used in the superfusion by programming of an Apple IIe computer. This system makes it possible to test various experimental groups in the same run (4 groups of 5 chambers). After a washout period of 45 min, at a flow rate of 0.5 ml/min, veratridine (40 μM) is added for 5 min to the superfusion medium. When drugs are tested (trimebutine and its stereoisomers), they are added to the superfusion medium 15 min before and also during veratridine application. Fractions of superfusate corresponding to 5 min are collected during the 30 min following the stimulation. At the end of the run, the slices are removed from the chambers and 2.5 ml of scintillation liquid (Hionic Fluor, Packard ) are added to the slices and to each of the fractions. The radioactivity is determined using liquid scintillation spectroscopy (Minaxi, Packard). The efflux of radioactivity is assumed to be due mainly to [3H]-glutamate efflux (Turner and Dunlap, 1989).

d) *Data analysis.*

All values are expressed as the mean ± SEM of at least 5 determinations. Release of radioactivity for each fraction is expressed in terms of fractional release calculated by dividing the radioactivity in each fraction by the amount remaining in the filter. The stimulation produced by veratridine is quantified by cumulating the release of radioactivity measured in the fractions collected after the stimulation. The effect of tested compounds is evaluated as percent of inhibition by comparing the total amounts of radioactivity released in control chambers to those released in chambers superfused with test compounds. From these percent inhibitions, $IC_{50}$ values are calculated by plotting probit values of inhibition versus log values of concentrations. Statistical analyses are performed using Student's unpaired two tailed t-test. Statistical differences are considered significant at $P < 0.05$.

2.2 Results

The results presented in Figure 2 demonstrate that trimebutine inhibited dose-dependently veratridine-induced glutamate release at concentrations higher than 60 µM (fig.2A). Furthermore, 50 to 60% inhibition could be reached at concentrations as high as 100 µM. (R)-trimebutine presented a profile similar to the racemate whereas (S)-trimebutine presented a significant inhibition from the concentration of 3 µM (fig.2A). The estimated $IC_{50}$ is 15.2 µM for (S)-trimebutine, whereas it could not be calculated ($IC_{50} > 100$ µM) for trimebutine and (R)-trimebutine. For Nor-TMB and its stereoisomers (fig.2B), the inhibitory effect was significant (p < 0.01) at 3, 10 and 30 µM and $IC_{50}$ value was 8.4 µM. (S)-Nor-TMB displayed an activity ($IC_{50} = 6.3$ µM) similar to that of the racemate and similar also to that of the second enantiomer (R )-Nor-TMB ($IC_{50} = 16.3$ µM). These results are in agreement with results from other papers reporting that compounds that inactivate voltage-dependent $Na^+$ channels prevent veratridine-induced glutamate release *in vitro* and *in vivo* (Lees and Leach, 1993). In a similar manner, the effect of TMB and related compounds on veratridine-induced glutamate release is due to their blocking activities on sodium channels.
When bupivacaine is evaluated under the same experimental conditions (Fig. 2C), an $IC_{50}$ value of 8.2 µM could be estimated.
Morphine is found inactive in this paradigm up to 100 µM (fig. 2C). The lack of effect of morphine in this model suggests that the effects of trimebutine on glutamate release are not due to the opioid properties of the compounds demonstrated in previous studies (Roman *et al.,* 1987).
These results demonstrate importantly that trimebutine displays higher activities than bupivacaine.
This example is quite important given the pivotal role of glutamate and excitatory amino acids (EAA) in the transmission of nociceptive message and more particularly in hyperalgesic conditions. In this respect, the finding that TMB and its metabolites are able of reducing the extracellular concentrations of glutamate by inhibiting its release from presynaptic pools represents an exciting property of TMB in its therapeutic use as analgesic agent.

**Example 3 : Electrophysiological experiments.**

The purpose of this example is the study of the effects of trimebutine and its stereoisomers on sodium currents.

3.1 Materials and methods

a) *DRG neurons.*

Experiments on sodium currents are performed using cultured rat dorsal root ganglia (DRG) excised from 14- to 15-day-old rat embryos. Methods for cell isolation and culture are derived from those described by Valmier *et al.* (1989). Pregnant Sprague-Dawley rats are killed by placing them in a $CO_2$ atmosphere for 5-6 min. Three to five embryos are removed aseptically and placed in a Petri dish containing the following B medium supplemented with antibiotics (streptomycin, 50 µg/ml; penicillin, 50 U/ml). The B medium contained (in mM): 137 NaCl, 5.4 KCl, 0.4 $Na_2HPO_4$, 0.8 $MgSO_4$, 0.8 $MgCl_2$, 1.8 $CaCl_2$, 6 glucose, 10 HEPES. The dorsal root ganglia are removed from the excised spinal cord and digested for 6 min in 2 ml of Dulbecco's modified Eagle medium containing 0.1% trypsin. Cells are dissociated mechanically through fire-polished Pasteur pipettes and plated in polyornithine-laminine coated dishes. The culture medium is the Neurobasal medium containing, 0.5 mM glutamine and 25 µM glutamate. The cells are incubated at 37°C in 5% $CO_2$. Electrophysiological experiments are performed from 4-6 h to 24 h after plating.

b) *Electrophysiology.*

Conventional whole cell patch clamp experiments are performed at room temperature using an EPC7 (List) patch clamp amplifier. DRG neurons are bathed in a Hanks derived medium containing (in mM): 143 NaCl; 10 $CaCl_2$;

5.6 KCl, 2 $MgCl_2$, 5 glucose and 10 HEPES, pH adjusted to 7.4 with NaOH (osmolarity, 300-310 mosm/1). For recording sodium current, calcium is replaced by $Mg^{2+}$ in the presence of 10 mM TEA (tetraethylammonium). Patch electrodes used for recording currents are filled with the following saline (in mM): 140 CsCl, 1.1 EGTA (ethyleneglycol-bis (β-aminoethyl ether) N, N, N', N'-tetraacetic acid), 5 HEPES, 2 $MgCl_2$, pH ajusted to 7.2 - 7.3 with CsOH (osmolarity, 290 mosm/1). The electrodes are pulled in 4 steps from 1.5 mM glass capillaries (GC 150 TF, Clark Electromedical Instruments) using a P87 puller (Sutter Instruments) and fire-polished. The tip resistance is 2-3 MΩ.

Drugs are dissolved in the bath medium (from stock solutions at $10^{-2}$ M in DMSO (dimethylsulfoxide)) and applied by pressure ejection (Pneumatic Picopump PV820, WPI) from glass pipettes (10 - 20 μm tip diameter) located at 50-60 μm from the recorded cell.

*c) Calculations.*

Data are sampled at 2 kHz. Software for stimulation, acquisition and analysis is constructed in house. The dose-response curves are constructed with various drug concentrations separated by wash periods. Each point is the mean ± SEM of 3 to 6 experiments.

Experimental points are fitted to the theoretical Hill curve using the least-square Minsq program: $y = 1 / (1 + [X]^n / IC_{50}^n)$ in which y is the fraction of $Na^+$ current persisting in the presence of the drug applied at the concentration [X], $IC_{50}$ is the concentration of drug that half-blocks the $Na^+$ current, and n is the Hill coefficient corresponding to the number of drugs required to block one $Na^+$ channel.

3.2 Results

In figure 3A are shown the effects of the successive 20 s applications of trimebutine at 0.1 and 1 μM on the sodium current of a DRG neuron. In this representative experiment, TMB induced a reversible blockade of the current amounting to 13% and 61% at 0.1 and 1 μM respectively. The blockade occurred without any evidence of changes in current kinetic (fig.3B) and voltage dependence (fig.3C). The dose-response curve obtained by applying 0.01, 0.1, 1 and 10 μM trimebutine is shown in figure 3(D) as a plot of the current part remaining in the presence of the blocker. The inhibition parameters calculated from this curve are : $IC_{50} = 1.05 \pm 0.09$ and $n_H = 1.09 \pm 0.10$. The parameters calculated for Nor-TMB are very similar.

A kinetic study is performed using (R,S)-TMB. The unblocking rate $k_{off}$ is determined from the time constant $\tau_{off} = 34 \pm 4$ s (n = 6) of the exponential recovery from block: $k_{off} = 1/\tau_{off} = 29 \ 10^{-3} \ s^{-1}$.

The blocking rate $k_{on}$ is deduced from $K_D = k_{off}/k_{on}$ ; $k_{on} = 35$-$40 \ 10^{-3} \ s^{-1} \ \mu M^{-1}$. These reaction rates defined the 3 drugs as fast $Na^+$ channel blockers; for instance, 10 μM (R,S)-TMB blocked the channels with a time constant of 2.2 s.

These electrophysiological data confirm the results on [3H]-batrachotoxin binding (example 1) and glutamate release (example 2) by demonstrating that TMB, its stereoisomers and its metabolites block reversibly the sodium currents in DRG neurons with $IC_{50}$ about 1 μM. Since the Hill coefficient is about 1, the blockade appeared to occur according to a simple bimolecular reaction, i.e. one molecule of blocker interacting with one site on the $Na^+$ channel. Therefore, the $IC_{50}$ value measured the dissociation constant $K_D$ of the blockers.

Hence, the effects of trimebutine and related compounds on $Na^+$ currents, which appears responsible for the inhibitory effect on glutamate release, indicates a potential therapeutic effect of these compounds in pain.

Sodium channel blockers like local anesthetics are known to block the generation and conduction of nerve impulses by inhibiting the current through voltage-gated $Na^+$ channels in the nerve cell-membrane (Strichartz and Ritchie, 1987). The effect of TMB and related compounds on $Na^+$ currents, which is probably responsible for the inhibitory effect on glutamate release, indicates a potential therapeutic effect of these compounds in pain.

**Example 4 : Corneal reflex testing in rabbits**

The aim of this example is to demonstrate the local anesthesic properties of trimebutine or its stereoisomers in comparison with a reference local anesthesic agent such as lidocaine.

4.1 Material and method

*a) Animals:*

White New-Zealand male rabbits (CEGAV, Les Hauts Noës, Saint Mars d'Egrenne, France) weighing 1.9-2.7 kg were used. Animals were housed individually in cages placed in an air-conditioned (17-21°C) animal house kept between 45% and 65% relative humidity and with an artificial day/night cycle (12h-12h) with light on at 7.30 am.

*b) Corneal reflex testing*

Test substances are administered as solutions in sterile water (Baxter, Maurepas, France). The concentrations of test substances are expressed as percentage (weight/volume) of active compound (base form). Each test substance at each concentration is tested on the two eyes of 5 animals (i.e. 10 measurements per substance and per concentration). On the day of the study, animals are placed in restraint cages in a quiet room. Twenty to 30 minutes later, a first measurement of the corneal sensitivity is performed. Any animal presenting a partial or complete corneal insensitivity or any ocular lesion before administration is excluded from the study. Immediately after the first measurement, 2 x 50 $\mu$l of test substances or their vehicle are applied to the cornea. The two instillations were undertaken on each eye at a one-minute interval. Corneal sensitivity is subsequently measured at 5, 10, 20, 30, 40, 50 and 60 minutes following the instillations, and then at 2, 3 and 4 hours after the instillations. Corneal sensitivity is measured by touching gently the center of the cornea with a loop-shape nylon yarn (0.3 mm diameter). For each measurement, this operation is repeated 10 times at regular 2 second intervals. The number of stimulations producing a corneal reflex is noted for each set and for each eye.

*c) Data analysis*

Results are expressed as percentage of inhibition of the corneal reflex calculated for each set of 10 stimulations:

% inhibition of corneal reflex = 10 - (number of stimulations inducing a corneal reflex) x 10

For each experimental group, median values are determined. The effects of test compounds are compared with those of the vehicle using a Mann-Withney non-parametric U test at each time of measurement. Areas under curves are calculated over the first 60-minute period following instillation for each animal and each concentration by the trapezoidal method of Bourget *et al.,* 1993. The relative potency of TMB is compared to that of lidocaine hydrochloride from respective mean areas under curves using a covariance analysis.

4.2 Results

TMB produced a dose-dependent local anesthetic effect on rabbit cornea (fig.4A). The first significant effects were obtained using the 0.1% concentration and lasted 20 min. An instillation of 0.3% or 1% TMB produced a complete local anesthesia lasting more than 60 min. Under the same experimental conditions, lidocaine (fig.4B) was found inactive up to the concentration of 0.3%. At the 6% concentration, the local anesthetic effect was complete only during 25 min and 60 min after the instillation, there was no more inhibition of the corneal reflex. When comparing areas under the curve, we calculated that TMB showed a potency 17-fold higher than lidocaine.

**Example 5 : PGE$_2$-induced hyperagesia**

The aim of this study is to evaluate the antihyperalgesic activity of TMB, its stereoisomers and its metabolites in combination with an opioid analgesic in Prostaglandin E$_2$ (PGE$_2$)-induced hyperalgesia in the rat.

*a) Animals:*

Test is carried out with Sprague-Dawley male rats (100-120 grams) on arrival. They are housed 5 per cage and acclimated to the conditions of the animal room for 5 days under a 12/12 day/night cycle and a constant room temperature of 22°C. Food and water are provided ad libidum.

*b) Test:*

A solution of PGE$_2$ (1mg/ml) is prepared as a stock solution in a 10% (v/v) alcohol in apyrogen sterile saline and stored at 4°C for 4 days.
A solution of PGE$_2$ (1$\mu$g/ml) is freshly prepared twice daily in sterile saline and 100$\mu$l is injected subplantar into the left paw of rat, twice daily for 4 days. Using this protocol, hyperalgesia is present for at least a week following completion for 4 days of treatment. Control animals (saline group) are given sterile saline in the same experimental conditions. Hyperalgesia is measured by the Randall and Selitto's test (Randall and Setillo,1957) using an analgesimeter (Ugo Basile).The analgesimeter is basically a device which exerts a force that increases at constant rate.The force is applied to the animal's paw which is placed on a small plinth under a cone-shaped pusher. The operator depresses a pedal-switch to start the mechanism with exerts the force. The nociceptive threshold is defined as the force at which the rat withdraws its paw. The threshold is determined before and after treatment. Drugs are given by subcutaneous route, 30 minutes before the second determination; Control animals (saline and PGE$_2$ group) received the appropriate vehicle in the same experimental conditions.

*c) Data analysis:*

Data are presented as the mean +/- SEM
The level of statistical significance is determined with Student's t test (Tallarrida and Murray, 1987) for paired sample and differences with $p < 0.05$ are considered statistically significant.
% of antinociceptive activity is calculated as follows:

$$\frac{\text{mean PGE}_2/\text{treated group} - \text{mean PGE}_2/\text{vehicle group}}{\text{after drug treatment} \qquad\qquad \text{after vehicle treatment}} \times 100$$

mean saline/vehicle group -      mean PGE$_2$/vehicle group

after vehicle group               after vehicle treatment

### Example 6 : Rat mononeuropathy

The aim of the study is to evaluate TMB, its stereoisomers and its metabolites in combination with an opioid analgesic in a model of rat neuropathy.
The Committee for Research and Ethical Issues of the International Association for the Study of Pain (IASP) Ethical Guidelines are adhered to in these studies. In particular, the duration of the experiments is as short as possible and the number of animals is kept to a minimum.

*a) Animals:*

Male Sprague-Dawley rats (Charles River, France, strain designation Crl :CD(SD)BR), n= 45, weighing 175-200 g on arrival are used. The rats are housed at the experimental facilities for a week prior to the experiments. They are maintained on a 12 h light/dark cycle and have free access to standard laboratory food and tap water in an ambient temperature of 20-22°C.

*b) Surgery:*

The unilateral peripheral mononeuropathy is produced on the right hind limb according to the method described by Bennett and Xie, 1988 and Attal et al., 1990. Rats are anesthetized with sodium pentobarbitone (Nembutal, 50 mg/kg i.p.). The common sciatic nerve is exposed by blunt dissection at the level of the mid-tigh; four ligatures (5-0 chromic catgut, about 1-mm spacing) are placed around the nerve.

*c) Antinociceptive testing:*

Experiments are carried out in a quiet room. Animals are not acclimatized to the test situations beforehand. The experimenter is unaware of the drug and doses used. Each animal receives drugs only once and is used in only one experiment. The antinociceptive action is determined by measuring the vocalization threshold elicited by pressure on both the nerve-injured and the contralateral hindpaw, using the Ugo Basile (Comerio, Italy) analgesymeter. This instrument generates a linearly increasing mechanical force applied by a dome-shaped plastic tip (diameter =1 mm) on the dorsal surface of the paw. The tip is positioned between the third and fourth metatarsus (into the sciatic nerve territory) and force is applied until the rat squeaked. For each rat, a control threshold (mean of two consecutive stable thresholds expressed in g) is determined before injecting the drugs . The vocalization thresholds are then measured every 10 min, until they returne to the level of the control values.

*d) Data analysis*:

Data are expressed as means ± S.E.M. The areas under the curves (AUC) are calculated using the trapezoidal rule. Statistical significance of the data is analysed by one-way analysis of variance (ANOVA). The observed significances are then confirmed with Tukey's test. Simple regressions (linear model) are performed to establish dose-dependent effects. Statistical analyses are carried out using a statistical computer program (Statgraphics Plus, Manugistics, Rockville, MD). P<0.05 is used as the criterion for statistical significance.

**References**

Allescher HD, Ahmad S, Classen M and Daniel EE (1991) Interaction of trimebutine and JO-1196 (fedotozine) with opioid receptors in the canine ileum. *JPharmacol Exp Ther* **257:** 836.-842.

Attal, N., Jazat, F., Kayser, V. and Guilbaud, G., Further evidence for 'pain-related' behaviours in a model of unilateral peripheral mononeuropathy, *Pain,* 41 (1990) 235-251.

Battaglia G and Rustioni A (1988) Coexistence of glutamate and substance P in dorsal root ganglion cells of the rat and monkey. *J Comp Neurol* **27**: 302-312.

Bean BP, Cohen CJ and Tsien RW (1983) Lidocaine block of cardiac sodium channels. *J Gen Physiol* **81**: 613-642.

Bennett, G.J. and Xie, Y.K., A peripheral mononeuropathy in rat that produces disorders of pain sensation like those seen in man [see comments], *Pain,* 33 (1988) 87-107.

Bourget P and Delouis JM (1993) Review of a technic for the estimation of area under the concentration curve in pharmacokinetic analysis. *Therapie* **48**: 1-5.

Bradette M, Delvaux M, Staumont G, Fioramonti G, Bueno L and Frexinos J (1994) Evaluation of colonic sensory thresholds in IBS patients using a barostat: definition of optimal conditions and comparison with healthy subjects. *Dig Dis Sci* **39**: 449-57.

Bueno L, Honde C, Pascaud X and Junien JL (1987) Effects of orally versus parenterally administered trimebutine on gastrointestinal and colonic motility in dogs. *Gastroenterol Clin Biol* **11**: 90B-93B.

Calcutt, N.A., Jorge, M.C., Yaksh, T.L. and Chaplan, S.R., Tactile allodynia and formalin hyperalgesia in streptozotocin-diabetic rats: effects of insulin, aldose reductase inhibition and lidocaine, *Pain,* 68 (1996) 293-299.

Cheng YC and Prusoff WH (1973) Relationship between the inhibition constant (Ki) and the concentration of inhibitor which causes 50 percent inhibition ($IC_{50}$) of an enzymatic reaction. *Biochem Pharmacol* **22**: 3099-4002.

Coderre TJ and Melzack R (1992) The contribution of excitatory amino acids to central sensitization and persistent nociception after formalin-induced tissue injury. *J Neurosci* **12**: 3665-3670.

Courteix, C., Eschalier, A. and Lavarenne, J., Streptozocin-induced diabetic rats: behavioural evidence for a model of chronic pain, *Pain,* 53 (1993) 81-88.

Delis C, Walker EA, Castillo FD, Evans DF, Wingate DL, Allouche S and Van Egroo LD (1994) The effect of stress and opioid agonist on postprandial motor activity in the human small bowel Digestive disease week (a) Abstract AGA, New Orleans, USA (b) *Gastroenterology* **106**: A 485.

Dickenson AH (1995) Spinal cord pharmacology of pain. *British Journal of Anesthesia.* **75**: 193-200.

Dray A, Urban L and Dickenson A (1994) Pharmacology of chronic pain. *Trends in Pharmacological Sciences* **15**: 190-197.

Frexinos J, Fioramonti J and Bueno L (1985) Effect of trimebutine on colonic myoelectrical activity in IBS patients. *Eur J Clin Pharmacol* **28**: 181 -185.

Ghidini O, Saponati G and Intrieri L (1986) Single drug treatment for irritable colon: rociverine versus trimebutine maleate. *Curr Ther Res* **39**: 541-548.

Grandjouan S, Chaussade S, Couturier D, Thierman-Duffaud D and Henry JF (1989) A comparison of metoclopramide and trimebutine on small bowel motility in humans. *Aliment Pharmacol Ther* **3**: 387-393.

Julia V, Coelho AM, Rouzade MI, Allouche S and Bueno L (1996) Influence de la trimébutine (Débridat) sur l'hypomotricité colique et les crampes abdominales liées à la distension rectale chez le rat. *Med Chir Dig* **25**: 239-242.

Lees G and Leach MJ (1993) Studies on the mechanism of action of the novel anticonvulsant lamotrigine (lamictal) using primary neuroglial cultures from rat cortex. *Brain Res.* **612**: 190-199.

Lüttecke K (1980) A three part controlled study of trimebutine in the treatment of irritable colon syndrome. *Cur Med Res Op* **6**: 437-443.

Mao J, Price DD, Hayes RL, Lu J, Mayer DJ and Frenk H (1993) Intrathecal treatment with dextrorphan or ketamine potently reduces pain-related behaviors in a rat model of peripheral mononeuropathy. *Brain Research* **605**: 164-168.

Mc Pherson GA (1985) Analysis of radioligand binding experiments: a collection of computer programs for the IBM PC. *JPharmacol Methods* **14**: 213-228.

Meert TF and Melis W (1992) Interactions between epidurally and intrathecally administered sufentanil and bupivacaine in hydroxypropyl-β-cyclodextrin in the rat. *Acta Anesthesiol Belg* **43**: 79-89.

Meunier P (1980) Effet de la trimébutine sur la motricité colique dans les colopathies.

Abstract, *Gastroenterol Clin Biol* **4**: 261A.

Moshal MG and Herron M (1979) A clinical trial of trimebutine in spastic colon. *J Int Med Res* **7**: 231-234.

Pascaud X, Roman F, Petoux F, Vauche D and Junien JL (1987) Action de la trimebutine sur la motricité gastro-intestinale. *Gastroenterol Clin Biol* **11**: 77B-81B.

Randall, L. and Selitto, J.L. (1957) Arch. Int. Pharmacodyn. , 4, 409-419

Rawal N (1990) Indications for the use of intraspinal opioids, in *Spinal Narcotics* (Rawal N and Coombs DW eds) pp 43-61, Kluwer Academic Publishers, Dordrecht.

Reboa G, Bertoglio C, Terrizzi A and Parodi E (1976) L'azione della trimebutina sull'attivita elettrica e manometrica del colon normale e patologico. *Riv Gastroenterol* **28:** 1-16.

Roman F, Pascaud X, Taylor JE and Junien JL (1987) Interactions of trimebutine with guinea pig opioid receptors. *J Pharm Pharmacol* **39**: 404-407.

Sanguinetti MC and Kass RS (1984) Voltage-dependent block of calcium channel current in the calf cardiac purkinje fiber by dihydropyridine calcium channel antagonists. *Circ Res* 55(3): 336-348.

Schang JC, Devroede G and Pilote M (1993) Effect of trimebutine on colonic function in patients with chronic idiopathic constipation: evidence for the need of a physiologic rather than clinical selection. *Dis Colon Rectum* **36**: 330-336.

Strichartz G and Ritchie J (1987) The action of local anesthesics on ion channels of excitable tissues in *Local Anesthesics Handbook of Experimental Pharmacology* (Strichartz G ed) pp 21-52, Springer-Verlag, Heidelberg.

Tallarida R. J and Murray R. B. (1987) Manual of Pharmacological Calculations with Computer programs

Taniyama K, Sano I, Nakayama S, Matsuyama S, Takeda K, Yoshihara C and Tanaka (1991) Dual effect of trimebutine on contractility of the guinea pig ileum via the opioid receptors. *Gastroenterology* **101**: 1579-1587.

Tomlinson, K.C., Gardiner, S.M., Hebden, R.A. and Bennett, T., Functional consequences of streptozotocin-induced diabetes mellitus, with particular reference to the cardiovascular system, *Pharmacol Rev,* 44 (1992) 103-150.

Toussaint J, Cremer M and Pintens H (1981) Etude en simple aveugle de trimébutine et de la mébévérine dans le côlon irritable et la dyspepsie *Acta Ther* **7**: 261-268.

Triggle DJ (1997) Stereoselectivity of drug action [review]. *Drug Discovery Today* **2**: 138-147.

Turner TJ and Dunlap K (1995) Prolonged time course of glutamate release from nerve terminals: relationship between stimulus duration and the secretory event. *J Neurochem* **64**: 2022-2033.

Urban L, Thompson SWN and Dray A (1994) Modulation of spinal excitability: co-operation between neurokinin and excitatory amino acid neurotransmitters. *Trends Neurosci* **17**(10): 432-438.

Valmier J, Simmoneau M and Boisseau S (1989) Expression of voltage-dependent sodium and transient potassium currents in an identified subpopulation of dorsal root ganglion cells acutely isolated from 12-day-old mouse embryos. *Pflügers Arch* **414:** 360-368.

Wermelskirchen D, Wilffert B and Peters TJ (1992) Veratridine-induced intoxication: an in vitro model for the characterization of anti-ischemic compounds. *Basic Clin Physiol Pharmacol* **3**: 293-321.

**Claims**

1.  A product comprising trimebutine [2-dimethylamino-2-phenylbutyl-3, 4, 5- trimethoxy-benzoate hydrogen maleate] or its corresponding stereoisomers and an opioid analgesic.

2.  A product according to claim 1 wherein compounds can be used simultaneously, separately or sequentially in the treatment and/or prevention of pain and/or nociception.

3.  A product according to claim 1 wherein the opioid analgesic is chosen among morphine, codeine, dihydrocodeine, diacethylmorphine, hydrocodone, hydromorphone, levorphanol, onyxmorphone, alfentanyl, buprenorphine, butorphanol, fentanyl, sulfentanyl, meperidine, methadone, nalbuphine, propoxyphene and pentazocine or acceptable salts thereof.

4.  A product according to claim 3 wherein the opioid analgesic is morphine or an acceptable salt thereof.

5.  A pharmaceutical composition comprising trimebutine [2-dimethylamino-2-phenylbutyl-3, 4, 5- trimethoxy-benzoate hydrogen maleate] or its corresponding stereoisomers and an opioid analgesic with at least one pharmaceutical carrier or excipient.

**6.** Use of trimebutine [2-dimethylamino-2-phenylbutyl-3, 4, 5- trimethoxy-benzoate hydrogen maleate] or its corresponding stereoisomers and an opioid analgesic for the preparation of a medicament to prevent and/or treat pain and/or nociception.

**7.** Use according to claim 6 for simultaneous, separate or sequential administration in the treatment and/or prevention of pain and/or nociception.

**8.** Use according to claim 6 for the preparation of a medicament to prevent and/or treat acute pain .

**9.** Use according claim 6 for the preparation of a medicament to prevent and/or treat inflammatory pain.

**10.** Use according claim 6 for the preparation of a medicament to prevent and/or treat chronic pain.

**11.** Use according to claim 6 wherein the trimebutine is administered at dosage between 50 to 900 mg/day and preferably between 300 to 600 mg/day.

**12.** Use according to claim 6 wherein the opioid analgesic is administered at dosage between 0.01 to 250 mg/day depending of the chosen opioid analgesic.

**13.** Use according to claim 6 wherein the morphine is administered at dosage between 2 to 220 mg/day.

**14.** Method for preventing and/or treating pain and/or nociception comprising administering trimebutine and an opioid analgesic to a patient in need thereof.

**15.** Method according to claim 14, wherein the administration of trimebutine and the opioid analgesic is simoultaneous, separate or sequential.

**16.** A process for the preparation of a pharmaceutical composition comprising trimebutine or its stereoisomers and an opioid analgesic which process comprises bringing trimebutine and the opioid analgesic into association with a pharmaceutically acceptable carrier or excipient.

FIGURE 1

FIGURE 2A

FIGURE 2B

FIGURE 2C

EP 1 138 330 A1

FIGURE 3

FIGURE 4 A

FIGURE 4 B

EP 1 138 330 A1

**European Patent Office**

## PARTIAL EUROPEAN SEARCH REPORT

which under Rule 45 of the European Patent Convention shall be considered, for the purposes of subsequent proceedings, as the European search report

Application Number

EP 99 12 5752

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | ROMAN F J ET AL: "PHARMACOLOGICAL PROPERTIES OF TRIMEBUTINE AND N-MONODESMETHYLTRIMEBUTINE" JOURNAL OF PHARMACOLOGY AND EXPERIMENTAL THERAPEUTICS,AMERICAN SOCIETY FOR PHARMACOLOGY AND,US, vol. 289, no. 3, 1999, pages 1391-1397, XP000985364 ISSN: 0022-3565 * the whole document * | 1-16 | A61K31/485 //(A61K31/485, 31:24) |

----

|  |  |  | **TECHNICAL FIELDS SEARCHED (Int.Cl.7)** |
|---|---|---|---|
|  |  |  | A61K |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 3 August 2001 | Leherte, C |

**European Patent Office**

**INCOMPLETE SEARCH
SHEET C**

Application Number

EP 99 12 5752

Although claims 14 and 15 are directed to a method of treatment of the human/animal body (Article 52(4) EPC), the search has been carried out and based on the alleged effects of the compound/composition.

_____

Claim(s) searched completely:
    3, 4, 13

Claim(s) searched incompletely:
    1, 2, 5-12, 14-16

Reason for the limitation of the search:

Present claims 1, 2, 5-12 and 14-16 relate to a compounds defined (inter alia) by reference to the following parameter: " opioid analgesic".
The use of these parameters in the present context is considered to lead to a lack of clarity within the meaning of Article 84 EPC. It is impossible to compare the parameters the applicant has chosen to employ with what is set out in the prior art. The lack of clarity is such as to render a meaningful complete search impossible. Consequently, the search has been restricted to those parts relating to the compounds individually structurally identified by name in the claims, with due regard to the therapeutic applications mentioned in the claims.